# EUROPEAN PATENT SPECIFICATION

(11) **EP 3 363 473 B1**
(45) Date of publication and mention of the grant of the patent: **13.09.2023**
(21) Application number: 18157308.0
(22) Date of filing: 19.02.2018
(51) Int. Cl.: B65B 9/213, A61L 2/20, B65B 55/10, A61L 2/18, B65B 51/30, B65B 55/02

(54) **A STERILIZATION SYSTEM FOR A MACHINE MANUFACTURING CONTAINERS FOR POURABLE FOODSTUFFS**
EIN STERISATIONSSYSTEM FÜR EINE MASCHINE ZUR HERSTELLUNG VON BEHÄLTERN FÜR FLÜSSIGE LEBENSMITTEL
UN SYSTÈME DE STERILSATION POUR UNE MACHINE PRODUISANT DE RECIPIENTS POUR DES ALIMENTS ALIMENTAIRES LIQUIDES

(30) Priority: 20.02.2017 IT 201700018507
(43) Date of publication of application: 22.08.2018
(73) Proprietor: IPI S.r.l., 06132 Perugia (PG) (IT)
(72) Inventor: BIRELLO, Fabio, 40135 Bologna BO (IT); CRISTOFORI, Alessandro, 40124 Ferrara FE (IT); VENTURA, Maurizio, 40053 Valsamoggia BO (IT); VITALI, Antonio, 40062 Molinella BO (IT); ZANETTI, Federico, 40011 Anzola dell'Emilia BO (IT); NEGRINI, Stefano, 40012 Calderara di Reno BO (IT)
(74) Representative: Frontoni, Stefano

(56) References cited:
- EP-A1- 1 050 468
- WO-A1-03/066444
- BE-A- 824 787
- CN-A- 101 985 318
- JP-A- H01 111 633
- US-A- 4 225 556

## Description

The present invention falls within the technical field of machines for producing aseptic containers. In particular, the invention relates to a sterilization system for a machine for producing containers for pourable foodstuffs.

These containers (for example used for products such as fruit juices, various kinds of sauces, etc.) are obtained starting from a continuous web of packaging material formed by a plurality of layers of different materials coupled to each other (generally paper, aluminum and heat-sealable plastic material, for example polyethylene), which is processed in a dedicated automatic machine.

In the machine for producing such containers, the web of packaging material, initially wound in a coil, is unwound and fed towards following stations. In particular, the machine generally comprises a tank (the so-called "bath") containing a liquid sterilizing substance (generally hydrogen peroxide) within which the web is passed.

The material web is then folded, closed longitudinally with respect to its extent (so as to form a sort of tube) and filled with the foodstuff at the so-called aseptic chamber arranged downstream of the bath.

The closed and filled web moves along an output path in which it leaves the aseptic chamber through an opening, then is transversely welded with respect to the respective extent at a welding station and finally is cut in order to separate the filled and closed containers.

Then, the containers can be taken to a subsequent station where their final shape is defined.

A sterilization step cyclically occurs at the aseptic chamber, generally by supplying air and hydrogen peroxide. During this sterilization step, the tube feed is stopped along the output path and resumed when the sterilization is complete.

In order to prevent the sterilizing agent from escaping towards the welding station once the feed along the output path is resumed, a seal which comes into contact with the tube walls at the outlet of the aseptic chamber is generally provided, in order to close the latter. However, this seal is insufficient to ensure that the peroxide does not escape from the aseptic chamber once the tube feed is resumed.

The sterilizing agent escaping from the aseptic chamber causes the components below the aseptic chamber (for example those arranged in the welding area) to be damaged and worn. Furthermore, if there is sterilizing agent outside the aseptic chamber this can be harmful in case an operator has to access the machine (for example for repair or maintenance work).

Document EP2578505 seeks to solve the above mentioned problems by arranging a suction station downstream of the hermetic seal and the aseptic chamber. Suction means act in the suction station to suck the air containing the hydrogen peroxide escaped from the aseptic chamber.

However, the solution of EP2578505 has some drawbacks. Firstly, the system proposed by this document does not guarantee in an optimal manner the complete removal of the sterilizing substance towards the welding zone.

Secondly, the aforesaid solution is structurally not very advantageous, since it negatively affects the overall dimensions of the machine, in that it requires a dedicated station to be arranged below the aseptic chamber.

Furthermore, the roughness of materials in contact with each other (material web and seal) can cause undesired depressed air flows between the tube and the seal, and can further cause the seal to wear out.

Finally, with the known solution mentioned above, the continuous dragging of the seal on the tube-like material web can cause the latter to lose stability, also after the sterilization step, when the tube feed towards the output path of the aseptic chamber is resumed.

Further prior art relating to web sterilisation with sealing of the aseptic chamber is shown in US4225556, EP1050468, WO 03/066444, CN 101985318, BE 824787, however, none of the documents disclose the temporary activation and inactivation of the seal to avoid wear of the seal and damage to the web.

Object of the present invention is to overcome the above mentioned drawbacks.

This object is achieved by proposing a sterilization system for a machine for producing pourable foodstuffs according to the appended claims.

Advantageously, the proposed system completely prevents the sterilizing agent from escaping from the aseptic chamber, also after the sterilization step, during the web feed.

Moreover, contrary to known solutions, not only there is no depressed air flow between the tube and the seal but also no wear of the seal.

Furthermore, the overall dimensions of the machine are not structurally affected by the proposed solution, as it does not require a dedicated station to be arranged below the aseptic chamber.

Specific embodiments of the invention and further advantages will be made clearer in the following description, with the aid of the attached drawings, in which:
- figure 1 is a schematic sectional view of a detail of the sterilization system according to the present invention, in a first operative situation;
- figure 2 is a view similar to that of figure 1, in which the system is in a second operative situation.

Referring to the attached figures, a sterilization system according to the present invention has been denoted with the reference number 1. The system 1 is designed to be applied to a machine for producing containers for pourable foodstuffs starting from a web N of packaging material.

This type of machine, as known, allows closed and filled containers to be obtained starting from a continuous web of packaging material which is generally unwound from a coil and fed to subsequent stations, in which it is processed in order to obtain single filled and sealed containers.

The system 1 comprises: an aseptic chamber 2, comprising in turn an inlet section 20 for allowing the web N to enter the aseptic chamber 2 itself, and an outlet section 21 disposed downstream of the inlet section 20, for allowing the web N to leave the aseptic chamber 2.

The system 1 further comprises: supplying means 3, which can be activated to supply a sterilizing agent (for example hydrogen peroxide and air) in the aseptic chamber 2; a seal 4 arranged at the outlet section 21 of the aseptic chamber 2; and air conveying means 5, for conveying away air containing the sterilizing substance.

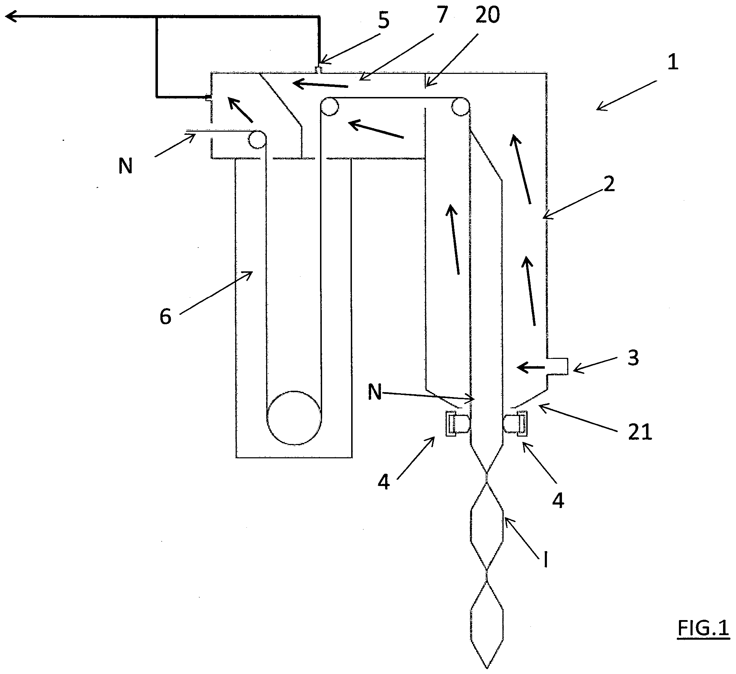

In particular, the seal 4 is designed so that it can be selectively activated in an operative configuration, in which it contacts the web N, for hermetically closing the outlet section 21 of the aseptic chamber 2 (see figure 1) when the supplying means 3 supply the sterilizing agent in the aseptic chamber 2. Thus, when the seal 4 is in the respective operative configuration, air can not pass between the web N and the seal 4 itself (therefore, no air can escape from the outlet section 21 of the aseptic chamber 2).

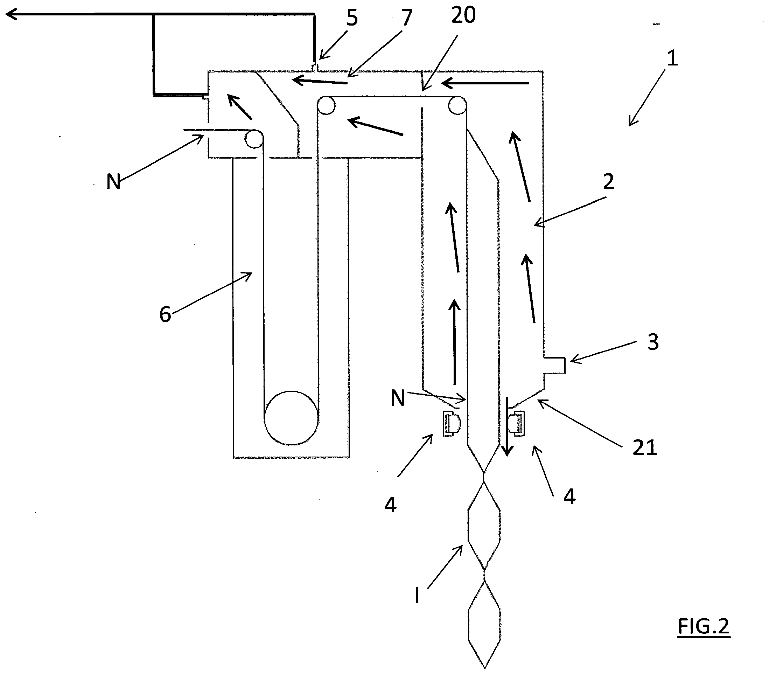

The seal 4 is also designed to be activated in an inoperative configuration, in which it is spaced from the web N, after the sterilizing agent is supplied in the aseptic chamber 2 (see figure 2). Thus, when the seal 4 is in the inoperative configuration, air can pass between the web N and the seal 4 itself (therefore, since the seal 21 does not occlude the outlet section 21 of the aseptic chamber 2 air can escape therefrom).

Furthermore, the air conveying means 5 are disposed upstream of the outlet section 21 of the aseptic chamber 2 and are arranged for conveying away air containing the residual sterilizing agent from the aseptic chamber 2 when the seal 4 is activated in its operative configuration.

In other words, during the supply of the sterilizing agent (together with air) in the aseptic chamber 2 by means of the supplying means 3, the seal 4 is in the respective operative configuration, i.e. it is in contact with the web N, which is stationary (i.e. the production operations of the machine are temporarily interrupted). Then, after the supply of the sterilizing agent, the air conveying means 5 remove air containing the residual sterilizing agent. Once this operation has been carried out, the seal 4 can be activated in the relative inoperative configuration, and the web N can continue its feed in the machine towards subsequent stations (i.e. it can leave the outlet section 21 of the aseptic chamber to move downstream thereof), further allowing the passage of air.

Therefore, in this way, advantageously, there is no risk that the sterilizing agent will undesirably escape downstream of the aseptic chamber 2 (for example towards the welding zone) since it is completely conveyed away upstream of the outlet section 21.

When the seal 4 is activated in the relative inoperative configuration, the supplying means 3 supply air in the aseptic chamber 2 without the sterilizing agent.

As shown in the figures, when the web N of packaging material comes into contact with the seal 4, it can be for example closed as a tube or can be in an "unfolded" configuration (situation not shown).

According to the preferred embodiment, the seal 4 comprises a variable volume chamber and lateral walls which delimit the chamber. In this case, preferably by means of suitable compression means (not shown herein), the chamber volume is filled with a fluid, such that the lateral walls of the seal 4 come into contact with the material web N when the seal 4 is activated in its operative configuration. In other words, the activation of the seal 4 in the relative operative configuration corresponds to the fluid being supplied within the seal chamber through the aforementioned compression means. The lateral walls of the seal 4 can be elastically deformable.

On the other hand, the activation of the seal 4 in the relative inoperative configuration corresponds to the fluid being drained from the seal chamber (i.e. the operation of allowing the fluid to escape from the chamber), preferably obtained by opening a valve (not shown) arranged in said chamber and which allows the fluid to escape from the chamber itself. In other words, the mentioned fluid is drained from the chamber volume through the controlled opening of said valve so that the lateral walls of the seal 4 are spaced from the material web N when the seal 4 is activated in the relative inoperative configuration.

Preferably, the fluid is compressed air. For example, the fluid is sterile compressed air. This ensures maximum sterility of the area adjacent to the aseptic chamber 2.

As an alternative, the seal may be movable in order to take the corresponding operative and inoperative configurations (situation not shown).

The seal 4 may have toroidal section.

As shown in the attached figures, the air conveying means 5 are preferably placed upstream of the inlet section 20 of the aseptic chamber 2. The air conveying means 5 comprise, for example, suction means and an air conduit.

Referring again to the figures, the system 1 further comprises a tank 6 containing a sterilizing substance (the so-called "bath", which comprises for example hydrogen peroxide), disposed upstream of the inlet section 20 of the aseptic chamber 2 and within which the web N passes before entering the aseptic chamber 2. The system 1 further comprises a connecting zone 7 (forming, for example, a conduit) which connects the tank 6 with the inlet section 20 of the aseptic chamber 2. In particular, in this embodiment, the air conveying means 5 are arranged at the connecting zone 7.

In this way, advantageously, the air conveying means generally already present at the tank 6 are used. In fact, as known, vapors which must be sucked, are generated from the tank 6 containing the sterilizing agent.

With specific reference to figure 1, in detail, it is possible to see the system 1 with the seal 4 in the relative operative configuration. In this figure, the arrows denote the air flow containing the sterilizing agent. In this situation, in fact, the sterilization of the web N disposed in the aseptic chamber 2 takes place with the web N being stationary.

In figure 2, on the other hand, the seal 4 is in the relative inoperative configuration. In this figure, the arrows denote the air flow without the sterilizing agent. In this situation, in fact, there is no sterilization of the web N but, on the contrary, the web N moves towards the outlet of the aseptic chamber, and the air can be output downstream of the aseptic chamber 2.

In both figures 1 and 2, the web N in the aseptic chamber 2 is closed as a tube and then filled with the foodstuff. Once the tube-like web N has left the aseptic chamber 2 through the outlet section 21, it is welded transversely to its extent by suitable welders (not shown), to form a series of filled and sealed wrappers I (schematically shown in figures) which are then separated by cutting blades (not shown).

In the appended figures the supplying means 3 are distal to the inlet section 20 of the aseptic chamber 2 and proximal to the outlet section 21 of the aseptic chamber 2. In this way, advantageously, a flow (of air and sterilizing agent or sterile air only) is created which goes from the bottom upwards, and then can be conveyed away by the air conveying means 5.

The invention further relates to a machine for producing containers for pourable foodstuffs, comprising a sterilization system 1 according to any one of the embodiments described above.

## Claims

1. A sterilization system (1) for a machine producing containers for pourable foodstuffs starting from a web (N) of packaging material, comprising:
an aseptic chamber (2), comprising an inlet section (20) for allowing the web (N) to enter the aseptic chamber (2), and an outlet section (21), disposed downstream of the inlet section (20), for allowing the web (N) to leave the aseptic chamber (2);
supplying means (3) configured to be activated for supplying a sterilizing agent into the aseptic chamber (2);
a seal (4), disposed at the outlet section (21) of the aseptic chamber (2);
air conveying means (5) for conveying away air containing the sterilizing agent, the air conveying means (5) are placed upstream of the outlet section (21) of the aseptic chamber (2);
**characterized in that**:
the seal (4) is designed so that it can be selectively activated in an operative configuration, in which it contacts the web (N) when the supplying means (3) supply the sterilizing agent in the aseptic chamber (2), for hermetically closing the outlet section (21) of the aseptic chamber (2); and in an inoperative configuration, in which it is spaced from the web (N), following the sterilizing agent supply in the aseptic chamber (2);
and **in that**:
the air conveying means (5) are configured for conveying away air containing the sterilizing agent from the aseptic chamber (2) when the seal (4) is activated in its operative configuration.

2. The system (1) according to claim 1, in which the seal (4) comprises a variable volume chamber and lateral walls delimiting the chamber; and in which when the seal (4) is activated in its operative configuration the chamber volume is filled with a fluid in such a way that the lateral walls of the seal contact the material web (N).

3. The system (1) according to the preceding claim, in which when the seal (4) is activated in its inoperative configuration the chamber volume is emptied of said fluid in such a way that the lateral walls of the seal are spaced from the material web (N).

4. The system (1) according to claim 2 or 3, in which the fluid is compressed air.

5. The system (1) according to any one of the preceding claims, in which the air conveying means (5) are placed upstream of the inlet section (20) of the aseptic chamber (2).

6. The system (1) according to the preceding claim, further comprising a tank (6) containing a sterilizing agent, placed upstream of the inlet section (20) of the aseptic chamber (2) and into which the web (N) passes before entering the aseptic chamber (2); and a connection zone (7) connecting the tank (6) with the inlet section (20) of the aseptic chamber (2); wherein the air conveying means (5) are placed at the connection zone (7).

7. The system (1) according to any one of the preceding claims, in which the supplying means (3) are distal from the inlet section (20) of the aseptic chamber (2) and proximal to the outlet section (21) of the aseptic chamber (2).

8. The system (1) according to any one of the preceding claims, in which the air conveying means (5) comprise suction means and at least an air conduit.

9. A machine for producing containers for pourable foodstuffs, comprising a sterilization system (1) according to any one of the preceding claims.

## Patentansprüche

1. Sterilisationssystem (1) für eine Maschine, die ausgehend von einer Bahn (N) von Verpackungsmaterial Behälter für gießbare Lebensmittel herstellt, umfassend:
eine aseptische Kammer (2), umfassend einen Einlassabschnitt (20) zum Ermöglichen, dass die Bahn (N) in die aseptische Kammer (2) eintritt, und einen Auslassabschnitt (21), der stromabwärts bezogen auf den Einlassabschnitt (20) angeordnet ist, zum Ermöglichen, dass die Bahn (N) die aseptische Kammer (2) verlässt;
Zuführmittel (3), dafür gestaltet, zum Zuführen eines Sterilisationsmittels in die aseptische Kammer (2) aktiviert zu werden;
eine Dichtung (4), die an dem Auslassabschnitt (21) der aseptischen Kammer (2) angeordnet ist;
Luftfördermittel (5) zum Wegbefördern von Luft, die das Sterilisationsmittel enthält, wobei das Luftfördermittel (5) stromaufwärts bezogen auf den Auslassabschnitt (21) der aseptischen Kammer (2) angeordnet ist;
**dadurch gekennzeichnet, dass**:
die Dichtung (4) so ausgelegt ist, dass sie selektiv in eine Arbeitskonfiguration, in der sie mit der Bahn (N) in Kontakt steht, wenn das Zuführmittel (3) das Sterilisationsmittel in die aseptische Kammer (2) zuführt, um den Auslassabschnitt (21) der aseptischen Kammer (2) hermetisch zu verschließen; und in eine Ruhekonfiguration, in der sie von der Bahn (N) beabstandet ist, nach dem Zuführen von Sterilisationsmittel in die aseptische Kammer (2), aktiviert werden kann;
und dass:
das Luftfördermittel (5) dafür gestaltet ist, Luft, die das Sterilisationsmittel enthält, von der aseptischen Kammer (2) weg zu befördern, wenn die Dichtung (4) in ihre Arbeitskonfiguration aktiviert ist.

2. System (1) gemäß Anspruch 1, wobei die Dichtung (4) eine Kammer mit variablem Volumen und Seitenwände, die die Kammer begrenzen, umfasst; und wobei, wenn die Dichtung (4) in ihre Arbeitskonfiguration aktiviert ist, das Kammervolumen mit einen Fluid gefüllt ist, so dass die Seitenwände der Dichtung mit der Materialbahn (N) in Kontakt stehen.

3. System (1) gemäß dem vorstehenden Anspruch, wobei, wenn die Dichtung (4) in ihre Ruhekonfiguration aktiviert ist, das Kammervolume von dem Fluid entleert ist, so dass die Seitenwände der Dichtung von der Materialbahn (N) beabstandet sind.

4. System (1) gemäß Anspruch 2 oder 3, wobei das Fluid Druckluft ist.

5. System (1) gemäß einem der vorstehenden Ansprüche, wobei das Luftfördermittel (5) stromaufwärts bezogen auf den Einlassabschnitt (20) der aseptischen Kammer (2) angeordnet ist.

6. System (1) gemäß dem vorstehenden Anspruch, ferner umfassend einen Tank (6), der ein Sterilisationsmittel enthält, stromaufwärts bezogen auf den Einlassabschnitt (20) der aseptischen Kammer (2) angeordnet, den die Bahn (N) durchläuft, bevor sie in die aseptische Kammer (2) eintritt; und eine Verbindungszone (7), die den Tank (6) mit dem Einlassabschnitt (20) der aseptischen Kammer (2) verbindet; wobei das Luftfördermittel (5) an der Verbindungszone (7) angeordnet ist.

7. System (1) gemäß einem der vorstehenden Ansprüche, wobei das Zuführmittel (3) fern von dem Einlassabschnitt (20) der aseptischen Kammer (2) und nahe dem Auslassabschnitt (21) der aseptischen Kammer (2) liegt.

8. System (1) gemäß einem der vorstehenden Ansprüche, wobei das Luftfördermittel (5) ein Saugmittel und wenigstens einen Luftkanal umfasst.

9. Maschine zur Herstellung von Behältern für gießbare Lebensmittel, umfassend ein Sterilisationssystem (1) gemäß einem der vorstehenden Ansprüche.

## Revendications

1. Système de stérilisation (1) pour une machine produisant des récipients pour des produits alimentaires liquides à partir d'une bande (N) de matériau d'emballage, comprenant :
une chambre aseptique (2), comprenant une section d'entrée (20) pour permettre à la bande (N) d'entrer dans la chambre aseptique (2), et une section de sortie (21), disposée en aval de la section d'entrée (20), pour permettre à la bande (N) de sortir de la chambre aseptique (2) ;
des moyens d'alimentation (3) configurés pour être activés afin de fournir un agent de stérilisation dans la chambre aseptique (2) ;
un joint d'étanchéité (4), disposé au niveau de la section de sortie (21) de la chambre aseptique (2) ; des moyens de transport d'air (5) pour évacuer l'air contenant l'agent de stérilisation, les moyens de transport d'air (5) étant placés en amont de la section de sortie (21) de la chambre aseptique (2) ;
**caractérisé en ce que** :
le joint d'étanchéité (4) est conçu de manière à pouvoir être activé sélectivement dans une configuration fonctionnelle, dans laquelle il est en contact avec la bande (N) lorsque les moyens d'alimentation (3) fournissent l'agent de stérilisation dans la chambre aseptique (2), pour fermer hermétiquement la section de sortie (21) de la chambre aseptique (2) ; et dans une configuration inactive, dans laquelle il est éloigné de la bande (N), après la fourniture de l'agent de stérilisation dans la chambre aseptique (2) ;
et **en ce que** :
les moyens de transport d'air (5) sont configurés pour évacuer l'air contenant l'agent de stérilisation de la chambre aseptique (2) lorsque le joint d'étanchéité (4) est activé dans sa configuration fonctionnelle.

2. Système (1) selon la revendication 1, le joint d'étanchéité (4) comprenant une chambre à volume variable et des parois latérales délimitant la chambre ; et, lorsque le joint d'étanchéité (4) est activé dans sa configuration fonctionnelle, le volume de chambre étant rempli d'un fluide de telle sorte que les parois latérales du joint d'étanchéité entrent en contact avec la bande de matériau (N).

3. Système (1) selon la revendication précédente, lorsque le joint d'étanchéité (4) est activé dans sa configuration inactive, le volume de la chambre étant vidé dudit fluide de telle sorte que les parois latérales du joint d'étanchéité sont éloignées de la bande de matériau (N).

4. Système (1) selon la revendication 2 ou 3, le fluide étant de l'air comprimé.

5. Système (1) selon l'une quelconque des revendications précédentes, les moyens de transport d'air (5) étant placés en amont de la section d'entrée (20) de la chambre aseptique (2).

6. Système (1) selon la revendication précédente, comprenant en outre un réservoir (6) contenant un agent de stérilisation, placé en amont de la section d'entrée (20) de la chambre aseptique (2) et dans lequel la bande (N) passe avant d'entrer dans la chambre aseptique (2) ; et une zone de raccordement (7) reliant le réservoir (6) à la section d'entrée (20) de la chambre aseptique (2) ; les moyens de transport d'air (5) étant placés au niveau de la zone de raccordement (7).

7. Système (1) selon l'une quelconque des revendications précédentes, les moyens d'alimentation (3) étant distaux de la section d'entrée (20) de la chambre aseptique (2) et proximaux de la section de sortie (21) de la chambre aseptique (2).

8. Système (1) selon l'une quelconque des revendications précédentes, les moyens de transport d'air (5) comprenant des moyens d'aspiration et au moins un conduit d'air.

9. Machine pour la production de récipients pour des produits alimentaires liquides, comprenant un système de stérilisation (1) selon l'une quelconque des revendications précédentes.
